(19) Europäisches Patentamt
European Patent Office
Office européen des brevets

(11) **EP 2 286 859 A1**

(12) **EUROPÄISCHE PATENTANMELDUNG**

(43) Veröffentlichungstag:
**23.02.2011 Patentblatt 2011/08**

(51) Int Cl.:
***A61M 5/36*** (2006.01)

(21) Anmeldenummer: **10008695.8**

(22) Anmeldetag: **20.08.2010**

(84) Benannte Vertragsstaaten:
**AL AT BE BG CH CY CZ DE DK EE ES FI FR GB
GR HR HU IE IS IT LI LT LU LV MC MK MT NL NO
PL PT RO SE SI SK SM TR**
Benannte Erstreckungsstaaten:
**BA ME RS**

(30) Priorität: **21.08.2009 DE 102009038495**

(71) Anmelder: **Medtron AG
66128 Saarbrücken (DE)**

(72) Erfinder: **Schweigmann, Michael
66332 Zweibrücken (DE)**

(74) Vertreter: **Müller-Gerbes Wagner Albiger
Patentanwälte
Friedrich-Breuer-Strasse 72-78
53225 Bonn (DE)**

(54) **Vorrichtung zur Detektion von Gasansammlungen**

(57) Die Erfindung betrifft eine Vorrichtung zur Detektion von Gasansammlungen in einer flüssigkeitsführenden Schlauchleitung (1), umfassend eine auf der Schlauchleitung angeordnete Manschette (2) und mindestens einen der Manschette zugeordneten Sensor (3a), mittels dessen Gasansammlungen in der Schlauchleitung detektierbar sind.

fig. 1

## Beschreibung

Die Erfindung betrifft eine Vorrichtung zur Detektion von Gasansammlungen in einer flüssigkeitsführenden Schlauchleitung, insbesondere im Applikationsfeld der Injektion von Flüssigkeiten, z.B. Kontrastmittel oder physiologische Kochsalzlösung.

Die Injektion von Flüssigkeiten, wie Kontrastmitteln, in einen Patienten im Zuge bildgebender Untersuchungsverfahren, wie Computertomographie oder Magnetresonanztomographie wird üblicherweise maschinell mit einem Injektor durchgeführt, der patientennah aufgestellt ist und die zu injizierende Flüssigkeit über eine Schlauchleitung in den Patienten injiziert. Da üblicherweise während des Injektionsvorganges keine Bedienungsperson sich in der Nähe des Injektors befindet und den Injektionsvorgang beaufsichtigen kann, besteht ein stetiger Bedarf an Sicherheitseinrichtungen, um die Injektion dahingehend zu überwachen, dass keine Gasansammlungen in der Schlauchleitung vorliegen, da die Injektion beispielsweise eines relevanten Luftvolumens in den Patienten leicht zu einer lebensbedrohlichen Situation führen kann.

Es sind daher bereits Vorrichtungen vorgeschlagen worden, bei denen versucht wird, eine leere, d.h. nicht mit Flüssigkeit gefüllte Spritze in einem Kolbeninjektor zu erkennen und einen Injektionsvorgang in diesem Fall zu blockieren, um zumindest das Risiko einer sogenannten Leerinjektion auszuschließen. Es kann jedoch beispielsweise bei unsachgemäßem Aufziehen der Spritze, welche in den Injektor eingelegt wird oder auch bei Undichtigkeiten im Schlauchsystem zu Gaseinschlüssen in der flüssigkeitsführenden Schlauchleitung kommen, die über eine Leerspritzenerkennung im vorgenannten Sinne nicht erkennbar ist, gleichwohl aber eine erhebliche Gefahr für den Patienten darstellt.

Auch ist bereits versucht worden, Luftblasen mittels Ultraschallsensoren zu erkennen. Diese Ausführungsform wird vorwiegend bei Rollenpumpen eingesetzt, benötigt aber einen festen Installationsort, bestimmte Schlauchmaterialien und ist sehr verschmutzungsanfällig, was verbesserungswürdig erscheint.

Aufgabe der Erfindung ist es daher, eine Vorrichtung zur Detektion von Gasansammlungen in einer flüssigkeitsführenden Schlauchleitung der eingangs genannten Art vorzuschlagen, die sich an die vorhandenen Untersuchungsgerätschaften und insbesondere die vorhandenen Injektoren einfach anpassen lässt und eine zuverlässige Detektion auch kleinerer Gasansammlungen in einer flüssigkeitsführenden Schlauchleitung gewährleistet.

Zur Lösung der gestellten Aufgabe wird erfindungsgemäß die Ausbildung einer Vorrichtung gemäß den Merkmalen des Patentanspruches 1 vorgeschlagen.

Vorteilhafte Ausgestaltungen und Weiterbildungen der Erfindung sind Gegenstand der abhängigen Ansprüche.

Die Erfindung schlägt vor, auf der Schlauchleitung eine Manschette anzuordnen, wobei der Manschette mindestens ein Sensor zugeordnet wird, mittels dessen die Gasansammlungen in der Schlauchleitung detektierbar sind. Die Manschette sitzt von daher beim erfindungsgemäßen Vorschlag auf der Schlauchleitung und kommt nicht mit der darin geführten Flüssigkeit in Kontakt und dient als Positionierungsmittel für den die flüssigkeitsführende Schlauchleitung im Hinblick auf etwaige Gasansammlungen überwachenden mindestens einen Sensor.

Um die im Rahmen der vorgesehenen Anwendung an einen Injektor für medizinische Untersuchungen herrschenden Anforderungen zu erfüllen, ist nach einem Ausführungsbeispiel der Erfindung der mindestens eine Sensor innerhalb der Manschette angeordnet, d.h. der mindestens eine Sensor ist in die Manschette integriert, von daher innerhalb der Manschette vor Beschädigung und Verschmutzung geschützt. Eine solche Manschette mit integriertem mindestens einen Sensor lässt sich auch leicht sterilisieren.

Nach einem Vorschlag der Erfindung ist die Manschette als Einmalartikel unmittelbar mit der üblicherweise ebenfalls als Einmalartikel ausgebildeten flüssigkeitsführenden Schlauchleitung verbunden. Hierbei kann vorgesehen sein, einen kurzen Teilabschnitt der flüssigkeitsführenden Schlauchleitung mit der erfindungsgemäß vorgeschlagenen Manschette und dem zugeordneten mindestens einen Sensor auszubilden, wobei dieser Abschnitt der Schlauchleitung mit endseitigen Anschlusselementen, beispielsweise Luer-Lock-Anschlüssen ausgebildet ist, um in ein an sich bekanntes System der flüssigkeitsführenden Schlauchleitung integriert zu werden. Alternativ ist es auch vorstellbar, die gesamte als Einwegartikel ausgebildete flüssigkeitsführende Schlauchleitung in der erfindungsgemäßen Weise an einer geeigneten Stelle mit einer Manschette und dem zugeordneten mindestens einen Sensor auszubilden.

Nach einem weiteren Vorschlag der Erfindung ist die Manschette lösbar auf der Schlauchleitung befestigbar, so dass sie nicht als Einwegteil, sondern als mehrfach verwendbare Einrichtung nutzbar ist und lediglich in an sich bekannter Weise die als Einwegartikel genutzte flüssigkeitsführende Schlauchleitung austauschbar ist. Im Vorfeld einer Untersuchung wird somit die erfindungsgemäß vorgeschlagene Manschette auf der flüssigkeitsführenden Schlauchleitung befestigt und nach Abschluss der Untersuchung zum Zwecke des Austausches der flüssigkeitsführenden Schlauchleitung von dieser abgenommen.

Nach einem Vorschlag der Erfindung kann eine solche lösbar auf der Schlauchleitung befestigbare Manschette in Längsrichtung der Schlauchleitung betrachtet etwa U-förmig mit U-Schenkeln ausgebildet sein, die einen über eine Einsecköffnung zugänglichen Aufnahmeraum für die Schlauchleitung begrenzen, dergestalt, dass die Manschette auf die Schlauchleitung aufsteckbar ist.

Der Vorteil einer lösbar auf der Schlauchleitung befestigbaren erfindungsgemäßen Manschette liegt neben der Mehrfachverwendbarkeit darin, dass sie an jeder beliebigen Stelle der flüssigkeitsführenden Schlauchleitung positioniert werden kann, beispielsweise möglichst nah am Patienten.

Nach einem weiteren Vorschlag der Erfindung ist die Manschette zweiteilig mit einem den mindestens einen Sensor umfassenden Oberteil und einem mit dem Oberteil über einen Steckverbinder verbindbaren Unterteil ausgebildet.

Während demgemäß im Oberteil der mindestens eine Sensor angeordnet ist und die Verbindung der Manschette mit der flüssigkeitsführenden Schlauchleitung auch im Bereich des Oberteiles der mehrteiligen Manschette bewirkt wird, kann das Unterteil dieser Manschette beispielsweise ein Elektronikmodul aufnehmen, welches Auswerteeinheiten für die Messergebnisse des mindestens einen Sensors umfasst und auch die Energieversorgung beispielsweise über einen im Unterteil aufgenommenen Akkumulator für den mindestens einen Sensor sicher stellen kann. Ein solches, einen Akkumulator zur Energieversorgung des mindestens einen Sensors aufnehmendes Unterteil kann zum Zwecke des Nachladens schnell und einfach gegen ein anderes Unterteil ausgetauscht werden.

Der mindestens eine Sensor der erfindungsgemäßen Vorrichtung kann als optischer oder elektrischer Sensor ausgebildet sein, wobei im Rahmen der Erfindung insbesondere auch die Kombination mehrerer optischer oder elektrischer Sensoren als auch die Kombination von optischen und elektrischen Sensoren, d.h. die Kombination unterschiedlicher Messverfahren vorgesehen sein kann.

Ein optischer Sensor kann beispielsweise mit sichtbarem Licht oder im Infrarotbereich arbeiten, wobei im Falle lediglich eines Sensors eine Reflektionsmessung erfolgen kann, die durch unterschiedliche Reflektion an den Grenzflächen von Schlauchwand, Gas und Injektionsmedium eine Detektion einer solchen Gasansammlung in der ansonsten flüssigkeitsführenden Schlauchleitung ermöglicht. Alternativ ist auch eine Durchleuchtungsmessung auf optischem Wege möglich, wozu mehrere Sensoren, nämlich mindestens ein Sender und mindestens ein Empfänger benötigt werden, die idealer Weise an gegenüberliegenden Seiten der flüssigkeitsführenden Schlauchleitung angeordnet werden und Änderungen des jeweiligen Brechungsindexes von Gas bzw. Flüssigkeit registrieren.

Ein nach elektrischen Verfahren arbeitender elektrischer Sensor führt bevorzugt eine Impedanzmessung durch, bei der je nach Anordnung der Sensoren der komplexe elektrische Widerstand quer durch die Schlauchleitung oder auch in Längsrichtung derselben bestimmt werden kann und insoweit Rückschlüsse auf das jeweils in der Schlauchleitung vorhandene Medium Flüssigkeit oder Gas gezogen werden können und Gasansammlungen detektierbar werden.

Bei Nutzung von optischen Sensoren ist es darüber hinaus auch möglich, nicht nur Gasansammlungen in der flüssigkeitsführenden Schlauchleitung zu detektieren, sondern darüber hinaus auch zu detektieren, ob überhaupt eine Schlauchleitung vorhanden ist, was insbesondere bei der lösbar auf der Schlauchleitung befestigbaren Variante der erfindungsgemäßen Manschette von Bedeutung ist. Ist nämlich die Manschette auf einer Schlauchleitung montiert, verändert sich die optische Strecke, was durch den optischen Sensor unmittelbar detektiert werden und an eine Steuereinrichtung gemeldet werden kann, um z.B. im Falle einer nicht auf der Schlauchleitung befestigten Manschette und damit fehlender Überwachung etwaiger Gasansammlungen den Injektionsvorgang zu blockieren.

Die von dem mindestens einen Sensor erzeugten Messwerte, die eine Unterscheidung zwischen Flüssigkeit und Gasansammlungen innerhalb der Schlauchleitung ermöglichen, können entweder drahtgebunden oder auch drahtlos über ein Funkmodul an eine Steuerungseinrichtung abgegeben werden.

Nach einem weiteren Vorschlag der Erfindung ist die Manschette aus einem Natur- oder Synthesekautschuk, beispielsweise Silikon hergestellt, der sich in der Medizin aufgrund seiner elastischen Materialeigenschaften und guter Temperatur und Chemikalienresistenz bewährt hat. In eine solche beispielsweise aus Silikon gefertigte Manschette kann der mindestens eine Sensor der erfindungsgemäßen Vorrichtung einfach integriert werden. Sofern optische Sensoren zur Anwendung kommen, ist die Manschette entsprechend lichtdurchlässig ausgebildet.

Weitere Ausgestaltungen und Einzelheiten der Erfindung werden nachfolgend anhand von Ausführungsbeispielen in der Zeichnung erläutert. Es zeigen:

Figur 1    eine erste Ausführungsform der erfindungsgemäßen Vorrichtung;

Figur 2    eine zweite Ausführungsform der erfindungsgemäßen Vorrich- tung;

Figur 3    eine dritte Ausführungsform der erfindungsgemäßen Vorrichtung;

Figur 4    eine vierte Ausführungsform der erfindungsgemäßen Vorrich- tung;

Figur 5    das Sensorprinzip bei den Ausgestaltungen gemäß Figuren 2 bis 4;

Figur 6    das Sensorprinzip bei der Ausgestaltung gemäß Figur 1;

Figur 7    eine fünfte Ausführungsform der erfindungsgemäßen Vorrich- tung;

Figur 8    eine sechste Ausführungsform der erfindungsgemäßen Vorrich- tung;

Fig. 9    in schematisierter Darstellung die kombinierte Anordnung von Sensoren im Rahmen einer erfindungsgemäßen Vorrichtung.

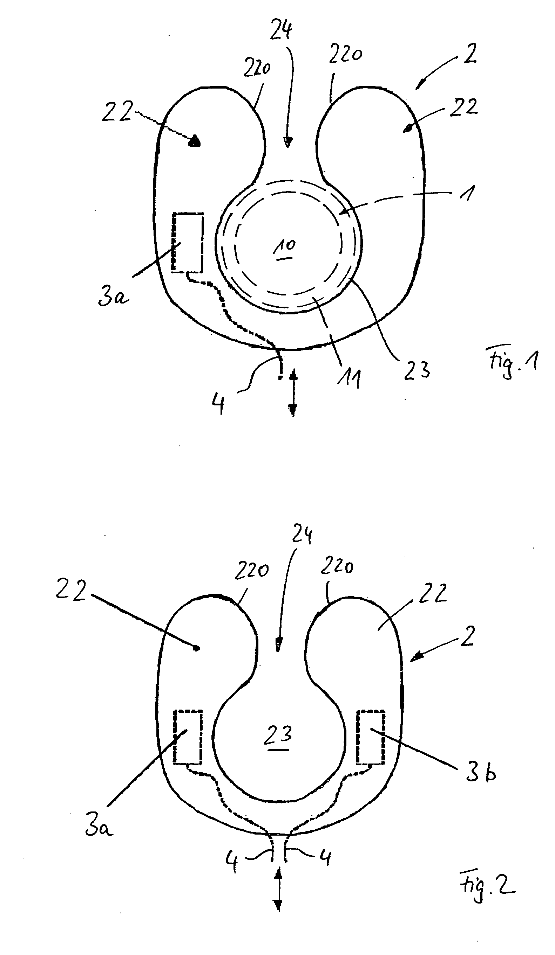

Aus der Figur 1 ist eine Vorrichtung zur Detektion von Gasansammlungen in einer flüssigkeitsführenden Schlauchleitung 1 ersichtlich, welche eine beispielsweise aus transparentem Silikon gefertigte Manschette 2 und einen der Manschette 2 zugeordneten, insbesondere in die Manschette 2 integrierten Sensor 3a umfasst, mittels dessen eine Gasansammlung in der Schlauchleitung 1 detektierbar ist, was nachfolgend noch näher erläutert wird.

Die Manschette 2 ist aus der aus der Figur 1 ersichtlichen Betrachtungsrichtung, welche der Längsrichtung der Schlauchleitung 1 entspricht, etwa U-förmig ausgebildet und weist zwei voneinander beabstandete U-Schenkel 22 auf, die eine Einstecköffnung 24 und einen über die Einstecköffnung 24 zugänglichen Aufnahmeraum 23 für die Schlauchleitung begrenzen. Der Aufnahmeraum 23 ist insoweit an den Durchmesser der Wandung 11 der Schlauchleitung 1 angepasst.

Bei der flüssigkeitsführenden Schlauchleitung 1 handelt es sich insbesondere um die von einem Injektor zu einem Patienten führende Schlauchleitung, um den Patienten während einer Untersuchung nach einem bildgebenden Verfahren, wie Computertomographie oder Magnetresonanztherapie eine Flüssigkeit, z.B. Kontrastmittel automatisiert zu injizieren.

Aufgrund der vorangehend geschilderten U-förmigen Konfiguration der Manschette 2 ist es möglich, diese durch Aufstecken an einer nahezu beliebigen Stelle der Schlauchleitung 1 zu befestigen, in dem die Schlauchleitung 1 durch die Einstecköffnung 24 hindurch in den Aufnahmeraum 23 eingeschoben wird, in welchem die Wandung 11 der Schlauchleitung 1 vollständig aufgenommen wird, so dass das Lumen 10 der Schlauchleitung 1 in vollem Querschnitt für die Führung von Flüssigkeit zur Verfügung steht.

Der innerhalb des gemäß der Darstellung der Figur 1 im linken U-Schenkel 22 angeordnete Sensor 3a ist vollständig in der Manschette 2 integriert, d.h. vor Beschädigung und Verschmutzung geschützt.

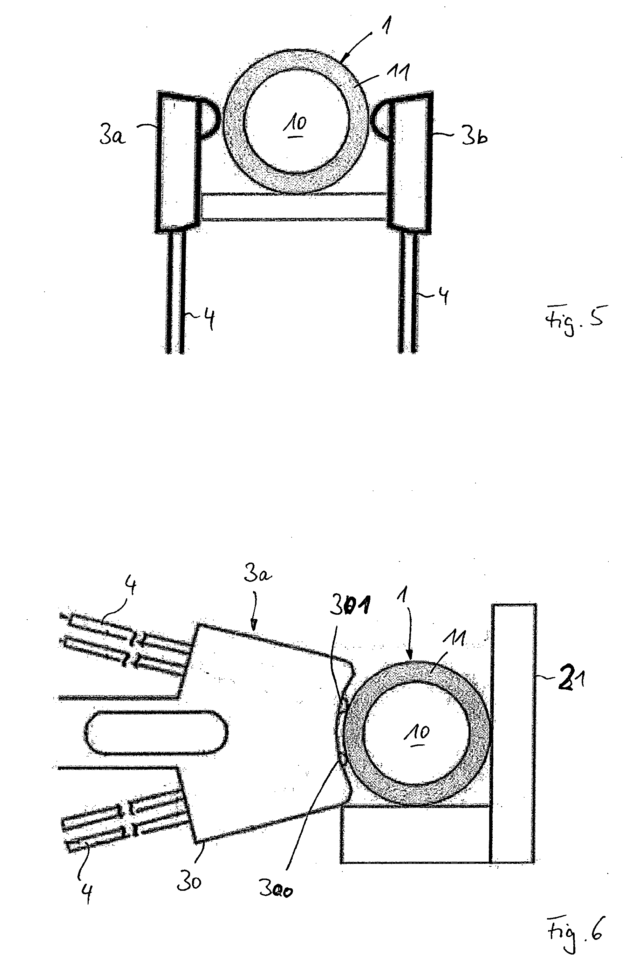

Der Sensor 3a ist als optischer Sensor ausgeführt und so ausgebildet, dass er eine auf die flüssigkeitsführende Schlauchleitung 1 gerichtete Reflexionsmessung durchführen kann, was in näheren Einzelheiten auch aus der Figur 6 ersichtlich ist.

Der optische Sensor 3a umfasst demgemäß einen Sender 300 und einen Empfänger 301, die in einem gemeinsamen Gehäuse 30 innerhalb der hier in Figur 6 nicht dargestellten Manschette 2 angeordnet sind und unter einem geeigneten Winkel auf die Wandung 11 der Schlauchleitung 1 gerichtet sind.

Das vom Sender 300 imitierte Licht, welches sowohl im sichtbaren wie auch im Infrarotbereich liegen kann, wird innerhalb der Schlauchleitung 1 reflektiert und trifft auf den Empfänger 301, der ein entsprechendes Messsignal generiert, welches über eine Signalleitung 4 einer hier nicht dargestellten Auswerteeinheit zugeführt werden kann. Die Signalintensität des vom Empfänger 301 empfangenden Lichts ändert sich jeweils spezifisch an den Grenzflächen der Schlauchwandung 11, etwaig innerhalb des Lumens 10 der Schlauchleitung 1 vorhandener Flüssigkeit oder eines Gaseinschlusses, so dass die Signalintensität sich bei Vorliegen von Gaseinflüssen gegenüber einer flüssigkeitsgeführten Schlauchleitung 1 ändert und vom Sensor entsprechende Messwerte generiert werden, die in einer nicht dargestellten Auswerteeinheit analysierbar sind. Die Auswerteeinheit kommuniziert zu diesem Zweck mit dem Sensor 3a über Signalleitungen 4.

Im Ausführungsbeispiel gemäß Figur 2 ist eine von dem Ausführungsbeispiel gemäß Figur 1 abweichende Ausgestaltung der Erfindung dargestellt, wobei hier wie auch in den nachfolgenden Ausführungsbeispielen gleiche Teile gleiche Bezugsziffern erhalten und zur Vermeidung von Wiederholungen nicht nochmals gesondert erläutert werden, sofern dies nicht zum Verständnis der Erfindung erforderlich ist.

Im Unterschied zum Ausführungsbeispiel gemäß Figur 1 ist im Ausführungsbeispiel gemäß Figur 2 nicht nur ein Sensor 3a vorgesehen, sondern es sind zwei Sensoren 3a, 3b innerhalb der Manschette 2 vorhanden, wobei jeweils ein Sensor 3a bzw. 3b in jeweils einen der beiden U-Schenkel 22 angeordnet ist. Hierbei sind die beiden Sensoren 3a, 3b unmittelbar gegenüberliegend und zwar im Bereich des größten Durchmessers des Aufnahmeraums 23 und damit auch der hier nicht dargestellten aufzunehmenden Schlauchleitung 1 angeordnet. Auch diese beiden Sensoren 3a, 3b sind als optische Sensoren ausgeführt, führen jedoch gemäß der Darstellung des Messprinzips in der Figur 5 eine Durchleuchtungsmessung der Schlauchleitung 1 durch, bei der die Messung der Signalintensität des durch die Schlauchleitung 1 hindurch laufenden Strahls gemessen wird. Hierzu ist beispielsweise der Sensor 3a als Sender und der Sensor 3b als Empfänger ausgebildet oder beide Sensoren 3a, 3b verfügen über jeweils einen Sender und einen Empfänger, die sich entsprechend wechselweise gegenüberliegen.

Die Charakterisierung von optisch durchsichtigen Medien erfolgt durch den sogenannten Brechungsindex bzw. die Brechzahl n, die als das Verhältnis der Lichtgeschwindigkeit c im Vakuum zur Lichtgeschwindigkeit $C_n$ im Medium mit der Brechzahl n definiert wird. Es gilt:

$$n = \frac{c}{c_n}$$

mit:

n = Brechzahl eines Mediums;

c = Lichtgeschwindigkeit im Vakuum;

$C_n$ = Lichtgeschwindigkeit eines Mediums mit der Brechzahl n.

Die Brechzahl von Wasser beträgt $n_{Wasser}$ = 1,33, während die von Luft mit $n_{Luft}$ = 1,0003 angegeben wird.

Trifft nun der Lichtstrahl des Senders 3a auf die Grenzfläche zweier Medien mit unterschiedlichen Brechzahlen, so wird nur ein Teil des einfallenden Lichts reflektiert und der andere Teil gelangt in das zweite Medium. Sofern die Brechzahl des zweiten Mediums größer ist als die des ersten, wird der Lichtstrahl zum Lot hin gebrochen, d.h. der Brechungswinkel verkleinert sich, ist hingegen die Brechzahl des zweiten Mediums kleiner als die des ersten vergrößert sich der Brechungswinkel. Dies wird Vorliegend ausgenutzt, da sich der Brechungswinkel und damit die vom Empfänger 3b empfangende Signalintensität der vom Sender 3a abgegebenen Lichtwellen verändert, wenn anstelle der gewünschten Flüssigkeit im Lumen 10 der Schlauchleitung ein Gaseinschluss beispielsweise in Form einer Luftblase strömt. Dies führt unmittelbar zu einer Änderung des über die Signalleitung 4 abgegebenen Messsignals, was in einer entsprechenden Steuereinrichtung zur Auslösung einer entsprechenden Warnung oder gar zur Abschaltung eines Injektors dienen kann.

Darüber hinaus verändert sich die optische Strecke, sobald eine Schlauchleitung 1 in der aus der Figur 1 ersichtlichen Weise in die Manschette 2 eingesetzt wird, so dass der wie vorangehend erläutert arbeitende optische Sensor 3a auch dazu genutzt werden kann, festzustellen, ob überhaupt eine Schlauchleitung 1 in die Manschette 2 eingelegt worden ist.

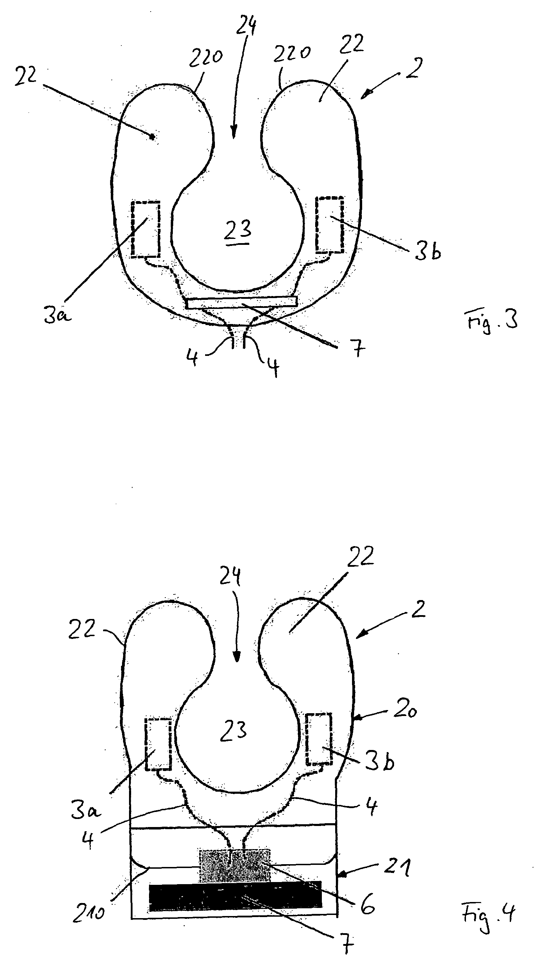

Im Ausführungsbeispiel gemäß Figur 3 ist in Ergänzung zum Ausführungsbeispiel gemäß Figur 2 angedeutet, dass innerhalb der Manschette 2 auch ein Elektronikmodul 7 angeordnet sein kann, welches beispielsweise eine mit den Sensoren 3a, 3b kommunizierende Auswerteeinheit für die Messsignale umfasst. Selbstverständlich können auch andere Einrichtungen im Elektronikmodul integriert werden.

Im Ausführungsbeispiel gemäß Figur 4 ist die Manschette 2 zweiteilig mit einem Oberteil 20 und einem Unterteil 21 ausgeführt, die über einen Steckverbinder 6 steckbar miteinander verbunden werden.

Im Oberteil 20 sind die Sensoren 3a, 3b angeordnet, während im Unterteil 21 das Elektronikmodul 7 beispielsweise mit Auswerteeinheit für die Sensoren 3a, 3b und gegebenenfalls auch mit einem Akkumulator zur kabellosen Stromversorgung der Sensoren 3a, 3b angeordnet ist. Ferner kann auch vorgesehen sein, ein Funkmodul (z.B. Zigbee) zur Kommunikation mit dem Injektor und/oder der Steuereinrichtung anzuordnen. In einer solchen Variante wird sodann lediglich eine drahtgebundene Injektoranbindung oder Anbindung zu einer Basisstation zum Aufladen des Akkumulators benötigt.

Bei einer Verschmutzung der Sensoren 3a, 3b kann im Ausführungsbeispiel gemäß Figur 4 die Steckverbindung zwischen Oberteil 20 und Unterteil 21 schnell und einfach aufgehoben werden und beispielsweise das die Sensoren 3a, 3b tragende Oberteil 20 ausgetauscht werden. Auch kann z.B. bei kritischem Akkuzustand innerhalb des Unterteils 21 lediglich das Unterteil zum Zwecke des Aufladens des Akkumulators gegen ein anderes Unterteil 21 ausgetauscht werden, ohne dass das Oberteil 22 von der im Aufnahmeraum 23 befindlichen Schlauchleitung 1 abgenommen werden muss. Die Aufladung des Unterteils kann entweder über eine Steckverbindung oder auch über eine kabellose Auflademöglichkeit, d.h. induktiv an einer Basisstation erfolgen, die z.B. in einem Injektor integriert sein kann.

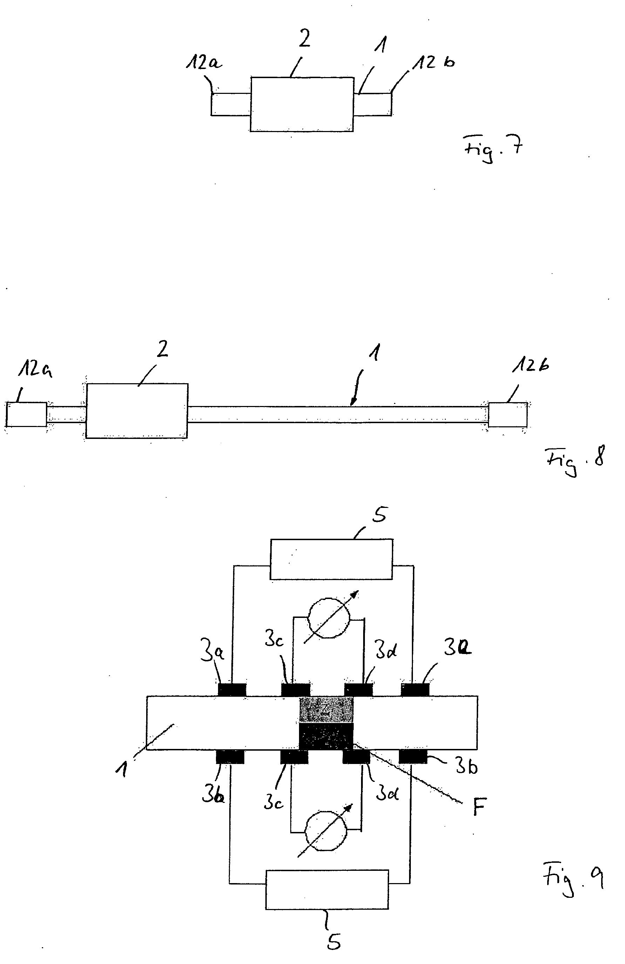

Die Ausführungsbeispiele gemäß Figuren 7 und 8 zeigen eine Abwandlung der vorangehend erläuterten Vorrichtungen, die nicht lösbar mit der Schlauchleitung 1 verbunden ist, sondern als Einmalartikel Bestandteil der Schlauchleitung 1 sind.

Im Ausführungsbeispiel gemäß Figur 7 ist die Manschette 2 mit den hier nicht dargestellten zugeordneten, vorzugsweise integrierten Sensoren auf einen kurzen Schlauchabschnitt der Schlauchleitung 1 montiert, die endseitig jeweils über Anschlusselemente, z.B. Luer-Lock-Anschlüsse 12a, 12b aufweist, um sie in eine vorhandene Schlauchleitung an einer geeigneten Stelle zu integrieren.

Im Ausführungsbeispiel gemäß Figur 8 ist eine Einweg-Schlauchleitung 1 dargestellt, die in Gänze die Verbindung zwischen dem Injektor und dem Patienten herstellt und bei der an geeigneter Stelle eine Manschette 2 mit zugeordnetem, vorzugsweise integrierten Sensoren vorgesehen ist.

Während bei den Ausführungsbeispielen gemäß Figuren 1 bis 4 jeweils optische Sensoren erläutert wurden, die die Ausbildung der Manschette 2 aus einem lichtdurchlässigen Material bedingen und überdies auch lediglich bei einer lichtdurchlässig ausgebildeten Schlauchwandung 11 der Schlauchleitung 1 zum Einsatz kommen können, nicht jedoch bei lichtundurchlässigen Gewebeschlauchleitungen 1, ist es auch möglich, elektrische Sensoren zu verwenden, die beispielsweise eine 2-, 3- oder 4-Punkt Impedanzmessung des komplexen elektrischen Widerstandes quer durch die Schlauchleitung 1 oder auch in deren Längsrichtung durchführen.

Gemäß dem Ausführungsbeispiel der Figur 9 kann auch eine kombinierte Anordnung mehrerer Sensoren 3a, 3b, 3c, 3d innerhalb einer einzigen Manschette 2 vorgesehen sein, die sowohl gleiche als auch unterschiedliche Messprinzipien anwenden.

So sind aus dem Ausführungsbeispiel der Figur 9 zwei mit Signalgeneratoren 5 in Verbindung stehende Elektroden 3a, 3b eines ersten Sensors ersichtlich sowie darüber hinaus Elektroden 3c, 3d eines zweiten Sensors, die gemeinsam eine Impedanzmessung in Längsrichtung der Schlauchleitung 1 durchführen. Die gemessene Impedanz ist vom Medium innerhalb der Schlauchleitung zwischen den Sensoren abhängig, so dass Gaseinschlüsse innerhalb der flüssigkeitsführenden Schlauchleitung 1 zuverlässig erkannt werden können und mittels der Sensoren überdies auch eine Messung dahingehend durchgeführt werden kann, ob überhaupt eine Schlauchleitung 1 in die Manschette 2 eingesetzt worden ist.

Die vorangehend erläuterten Vorrichtungen, die entweder mit einem optischen und/oder elektrischen Verfahren arbeiten, sind somit mindestens in der Lage, folgende Zustände zu erkennen:

• Eingelegte Schlauchleitung mit Luft
• Eingelegte Schlauchleitung mit Flüssigkeit (z.B. Kontrastmittel/physio• logische Kochsalzlösung)
• Kein Schlauch eingelegt
• Defekt/Verschmutzung der Vorrichtung

Es versteht sich, dass die Anzahl der Sensoren variabel gewählt werden kann und lediglich von den Platzverhältnissen innerhalb der Manschette begrenzt wird.

Aufgrund der Anordnung der Sensoren innerhalb einer Manschette wird eine hohe Passgenauigkeit und dadurch eine Verringerung der möglichen Sensorverschmutzung und Erhöhung der Sensorzuverlässigkeit erreicht.

Die erfindungsgemäße Vorrichtung wird, wenn nötig, für verschiedene Schlauchdicken in unterschiedlichen Größen gefertigt, z.B. für Durchmesser der Schlauchleitungen im Bereich zwischen 2 - 4 mm oder im Bereich zwischen 3 - 8 mm usw.

Die Manschette mit den darin zugeordneten Sensoren weist ein äußerst geringes Gewicht auf, so dass keine übermäßige Belastung und kein Zug auf den Schlauch ausgeübt wird.

**Patentansprüche**

1. Vorrichtung zur Detektion von Gasansammlungen in einer flüssigkeitsführenden Schlauchleitung (1), umfassend eine auf der Schlauchleitung

   (1) angeordnete Manschette (2) und mindestens einen der Manschette
   (2) zugeordneten Sensor (3a, 3b, 3c, 3d), mittels dessen Gasansammlungen in der Schlauchleitung (1) detektierbar sind.

2. Vorrichtung nach Anspruch 1, **dadurch gekennzeichnet, dass** der mindestens eine Sensor (3a, 3b, 3c, 3d) innerhalb der Manschette (2) angeordnet ist.

3. Vorrichtung nach Anspruch 1 oder 2, **dadurch gekennzeichnet, dass** die Manschette (2) lösbar auf der Schlauchleitung (1) befestigbar ist.

4. Vorrichtung nach Anspruch 3, **dadurch gekenn-**

**zeichnet, dass** die Manschette (2) in Längsrichtung der Schlauchleitung (1) betrachtet etwa U-förmig mit U-Schenkeln (22) ausgebildet ist, die einen über eine Einstecköffnung (24) zugänglichen Aufnahmeraum (23) für die Schlauchleitung (1) begrenzen, dergestalt, dass die Manschette (2) auf die Schlauchleitung (1) aufsteckbar ist.

5. Vorrichtung nach einem der Ansprüche 1 bis 4, **dadurch gekennzeichnet, dass** die Manschette (2) zweiteilig mit einem den mindestens einen Sensor (3a, 3b, 3c, 3d) umfassenden Oberteil (20) und einem mit dem Oberteil (20) über einen Steckverbinder (6) verbindbaren Unterteil (21) ausgebildet ist.

6. Vorrichtung nach einem der Ansprüche 1 bis 5, **dadurch gekennzeichnet, dass** die Manschette (2) eine mit dem mindestens einen Sensor (3a, 3b, 3c, 3d) kommunizierende Auswerteeinheit umfasst.

7. Vorrichtung nach einem der Ansprüche 1 bis 6, **dadurch gekennzeichnet, dass** der mindestens eine Sensor (3a, 3b, 3c, 3d) als optischer oder elektrischer Sensor ausgebildet ist.

8. Vorrichtung nach einem der Ansprüche 1 bis 7, **dadurch gekennzeichnet, dass** die Messwerte des mindestens einen Sensors (3a, 3b, 3c, 3d) drahtgebunden oder drahtlos an eine Steuerungseinrichtung abgebbar sind.

9. Vorrichtung nach einem der Ansprüche 1 bis 8, **dadurch gekennzeichnet, dass** die Manschette mit einer Stromquelle zur Stromversorgung des mindestens einen Sensors (3a, 3b, 3c, 3d) versehen ist.

10. Vorrichtung nach einem der Ansprüche 1 bis 9, **dadurch gekennzeichnet, dass** die Manschette aus Natur- oder Synthesekautschuk gefertigt ist.

fig. 1

fig. 2

Fig. 3

Fig. 4

Fig. 5

Fig. 6

Fig. 7

Fig. 8

Fig. 9

| Europäisches Patentamt | EUROPÄISCHER RECHERCHENBERICHT | Nummer der Anmeldung |
|---|---|---|
| European Patent Office Office européen des brevets | | EP 10 00 8695 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2008/098798 A1 (RILEY TIMOTHY A [US] ET AL) 1. Mai 2008 (2008-05-01) * Absätze [0054] - [0074]; Abbildungen 1A-1D * * Absätze [0083], [0084], [0086], [0088], [0091], [0098]; Abbildungen 4,5,7,8,10,15 * ----- | 1-10 | INV. A61M5/36 |
| X | WO 02/084256 A1 (ZEVEX INC [US]) 24. Oktober 2002 (2002-10-24) * Seite 3, Zeilen 8-30 * * Seite 7, Zeile 32 - Seite 8, Zeile 17; Abbildung 1 * * Seite 15, Zeile 25 - Seite 17, Zeile 24; Abbildungen 6,6A,6B,7,7A * ----- | 1-10 | |
| X | WO 2004/020992 A1 (FRESENIUS MEDICAL CARE DE GMBH [DE]; SCHNEIDER JOCHEN [DE]; SPENGLER G) 11. März 2004 (2004-03-11) * Seite 6 - Seite 8; Abbildungen * ----- | 1-4,10 | |
| A | EP 1 762 263 A1 (HOFFMAN LA ROCHE AG [CH]; ROCHE DIAGNOSTICS GMBH [DE]) 14. März 2007 (2007-03-14) * Absätze [0004], [0015], [0032]; Abbildungen * ----- | 1,6-9 | RECHERCHIERTE SACHGEBIETE (IPC) A61M |

Der vorliegende Recherchenbericht wurde für alle Patentansprüche erstellt

| Recherchenort | Abschlußdatum der Recherche | Prüfer |
|---|---|---|
| München | 25. November 2010 | Rosenblatt, Thomas |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
 
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 10 00 8695

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-11-2010

| Im Recherchenbericht angeführtes Patentdokument | Datum der Veröffentlichung | Mitglied(er) der Patentfamilie | Datum der Veröffentlichung |
|---|---|---|---|
| US 2008098798 A1 | 01-05-2008 | EP 2079494 A2<br>JP 2010508518 T<br>US 2009293588 A1<br>US 2008134750 A1<br>WO 2008051998 A2 | 22-07-2009<br>18-03-2010<br>03-12-2009<br>12-06-2008<br>02-05-2008 |
| WO 02084256 A1 | 24-10-2002 | AT 425447 T<br>AT 482386 T<br>CN 1494653 A<br>CN 1715866 A<br>DK 1381843 T3<br>EP 1381843 A1<br>EP 2040056 A2<br>ES 2323409 T3<br>JP 4156380 B2<br>JP 2004530473 T<br>US 2003141468 A1<br>US 6531708 B1 | 15-03-2009<br>15-10-2010<br>05-05-2004<br>04-01-2006<br>29-06-2009<br>21-01-2004<br>25-03-2009<br>15-07-2009<br>24-09-2008<br>07-10-2004<br>31-07-2003<br>11-03-2003 |
| WO 2004020992 A1 | 11-03-2004 | AU 2003260272 A1 | 19-03-2004 |
| EP 1762263 A1 | 14-03-2007 | KEINE | |

EPO FORM P0461

Für nähere Einzelheiten zu diesem Anhang : siehe Amtsblatt des Europäischen Patentamts, Nr.12/82